# EUROPEAN PATENT SPECIFICATION

(11) **EP 1 946 075 B1**
(45) Date of publication and mention of the grant of the patent: **06.03.2013**
(21) Application number: 06825885.4
(22) Date of filing: 12.10.2006
(51) Int. Cl.: A61L 2/20, A61L 2/24

(54) **METHOD AND APPARATUS FOR LOW ENERGY VAPORIZATION OF LIQUID OXIDIZING AGENTS OR SOLUTIONS**
VERFAHREN UND VORRICHTUNG ZUR NIEDRIGENERGETISCHEN VERDAMPFUNG FLÜSSIGER OXIDATIONSMITTEL ODER -LÖSUNGEN
PROCEDE ET DISPOSITIF DE VAPORISATION A ENERGIE LIMITEE, DE SOLUTIONS OU D'AGENTS D'OXYDATION LIQUIDES

(30) Priority: 31.10.2005 US 263331
(43) Date of publication of application: 23.07.2008
(73) Proprietor: GEA Lyophil GmbH, 50354 Hürth (DE)
(72) Inventor: FRINKE, George, D-35075 Gladenbach (DE); GROSS, Ralph, D-53227 Bonn-oberkassel (DE); KLUETSCH, Hubert, D-50733 Koeln (DE); MCDONNELL, Gerald, Basingstoke Hampshire RG21 3DP (GB)
(74) Representative: von Kreisler Selting Werner
(86) International application number: PCT/US2006/040025
(87) International publication number: WO 2007/053282

(56) References cited:
- US-A- 5 445 792
- US-A- 5 872 359
- US-A- 5 872 359
- US-A- 6 163 979
- US-A1- 2004 182 855
- US-B1- 6 564 471

## Description

### FIELD OF THE INVENTION

The present invention relates to a method and apparatus capable of vaporizing liquid oxidizing agents or solutions such as hydrogen peroxide and peracetic acid. The vaporization of oxidizing agents or solutions is performed in a vaporizer that preferably is evacuated to a very low pressure. The vaporized oxidizing agents or solutions are transferred to a sterilization vessel that preferably is evacuated to a very low pressure thereby allowing for the substantial homogenous distribution of the vaporized oxidizing agents or solution. The vessel contains a pressure sensor for detecting the partial pressure of the vaporized oxidizing agent. A microprocessor is programmed to terminate flow of the vaporized oxidizing agent to the vessel by closing a flow control valve which admits a liquid oxidizing agent or solution to the vaporizer. The apparatus is based upon a parametric system whereby at a predetermined partial pressure level of the vaporized oxidizing agent in the vessel, further vapor admitted thereto is terminated so as to prevent condensation of the oxidizing agent which can be harmful to the vessel and wasteful with regard to the utilization of excess oxidizing agent.

### BACKGROUND OF THE INVENTION

Conventional methods for dispersing gaseous oxidizing agents and the like are generally based on vaporizer designs that produce and distribute the gaseous oxidizing agent through use of a carrier gas which is generally filtered air or nitrogen. Thus, the amount of oxidizing gas contacting any exposed equipment surface, article, etc., contaminated with germs, bacteria, etc., is limited and the gas, which is generally dispersed at temperatures above ambient, upon contacting colder surfaces such as that of equipment or the walls of a sterilization vessel can undesirably form a condensate. The same is undesirable inasmuch as it is often detrimental to surfaces, can pose safety risks, differs in antimicrobial efficacy and the like. This risk can be minimized by blowing the oxidizing agent such as vaporized hydrogen peroxide (VHP) into the sterilization vessel as by pulsing. For optimal sterilization results, a high VHP concentration near its dew point is desired but the same requires accurate control of the amount of the VHP introduced into the vessel to prevent condensation.

U.S. Reissue Patent 33,007 relates to a method of vaporizing a multicomponent liquid, such as a hydrogen peroxide and water solution, for injection into a vacuum chamber including the steps of metering successive predetermined increments of the liquid at a predetermined rate onto a heated surface in a vaporization chamber. Upon exposure to the heated surface, each liquid increment is substantially instantaneously vaporized before the next succeeding liquid increment is metered onto the heated surface to produce a multi-component vapor increment having substantially the same weight percent composition as the multicomponent liquid increment. Each vapor increment is passed into the vacuum chamber.

U.S. Patent 5,527,508 relates to a method of enhancing penetration of a low vapor pressure sterilant vapor during sterilization of an article shaped to define a narrow opening, comprising the steps of (a) evacuating a closed chamber containing the article to a pressure below atmospheric pressure; (b) introducing only a sterilant vapor into the closed chamber in an amount effective to raise the pressure in the chamber to a predetermined second sub-atmospheric pressure; (c) allowing the introduced amount of sterilant vapor to diffuse throughout the closed chamber and into the article for a predetermined period of time which is less than or equal to twice the half-life of the sterilant vapor in the chamber; (d) introducing a compression gas into the closed chamber in an amount effective to raise the pressure in the chamber to a third subatmospheric pressure in a compression time period, wherein the third pressure is substantially greater than the second pressure and wherein the pressure differential between the third pressure and the second pressure is effective to drive the diffused sterilant vapor further into the article than the vapor has diffused such that the sterilant vapor substantially penetrates the article; and (e) repeating steps (a) through (d) until sterilization of the article is achieved.

The J. W. Johnson et al article Vaporized Hydrogen Peroxide Sterilization of Freeze Dryers, Technology/Applications, Vol. 46, No. 6 (12/1992) relates to the feasibility of using vapor hydrogen peroxide (VHP™) as an alternative to steam sterilization has been examined using a pilot plant freeze dryer equipped with a prototype vapor generator.

U.S. Patent 5,837,193 relates to a method of decontaminating or sterilizing freeze dryers at low temperature and pressure levels by utilizing sterilant vapor is disclosed.

U.S. Patent 5,872,359 relates to a system and a method for maintaining a selected concentration of a sterilant vapor during vapor phase sterilization. The system includes a sterilization chamber with a source of multicomponent vapor containing a sterilant vapor, an electromagnetic radiation source capable of generating radiation at a plurality of wavelengths, a radiation detector for detecting and quantitating the electromagnetic radiation absorbed at the wavelengths, the detector generating absorbance signals proportional to the concentration of the sterilant vapor in the multicomponent vapor, a microprocessor programmed to compare the absorbance signals with reference absorbance signals, to calculate the sterilant vapor concentration therefrom and to generate an output signal, and a controller to receive the output signal and to control addition of the sterilant vapor to the sterilization chamber to maintain a selected concentration of the sterilant vapor in the sterilization chamber during the sterilization process. The method allows sterilization conditions to be optimized to reduce the time required for and to increase the sterilization efficacy of the sterilization procedure.

U.S. Patent 6,875,399 relates to a sterilization system that includes a gas sensor for detection of a component, such as hydrogen peroxide vapor, in a multi-component vapor, such as a mixture of vapor hydrogen peroxide and water supplied to a chamber of the system. The sensor preferably uses a wavelength range in which hydrogen peroxide strongly absorbs but other components of the vapor, such as water, do not. A suitable wavelength for detection of hydrogen peroxide is from about 7500 to about 8400 nm, since there is little absorption by water in this range. This avoids the need to use complex subtraction procedures normally used to remove the contribution of water from detected absorbance measurements. A control system controls operating conditions of the sterilization system, such as a heater, pressure release valves, vaporization rate of hydrogen peroxide by a vaporizer, and the like to maintain optimum sterilization conditions within the chamber.

U.S. Patent 6,163,979 relates to a method for controlling a freeze drying process, wherein a frozen product is arranged on temperature controlled surfaces in an air-evacuated chamber and undergoes a main drying and after-drying phase. During the main drying phase, the temperature of the ice surrounding said product is continuously measured. The pressure in the chamber and/or the temperature of the surfaces are modified during transition from the main drying phase to the after-drying phase. In order to avoid longer idle periods between the chamber and the evacuation device and to determine transition from the main drying phase to the after-drying phase, the pressure and/or the temperature of the surfaces during said transition are modified according to changes in the temperature of the ice.

U.S. Patent 6,906,296 relates to a vaporizer heating apparatus comprised of electromagnetically responsive material and electrically non-conductive material. An antimicrobial fluid to be vaporized, such as water or hydrogen peroxide solution, is supplied to the heating apparatus where it is converted to a vapor. In one embodiment of the invention, electromagnetically responsive material particulate is embedded into the electrically non-conductive material. In another embodiment of the invention, a microwave generator is used to produce heat.

US 2004/0182855 A1 discloses a vaporizer heating apparatus being comprised of electromagnetically responsive material and electrically non-conductive material. An anti-microbial fluid to be vaporized, such as water or hydrogen peroxide solution, is supplied to the heating apparatus where it is converted to a vapor.

US 5445792 discloses a sterilization method which includes the steps of measuring the pressure within a sterilization chamber injecting a sterilant vapor into a flow of carrier gas, introducing the sterilant vapor and carrier gas into and through a sterilization chamber, and adjusting the rate of sterilant vapor injection to reach a pre-determined percentage of saturation limit for the sterilant vapor in the chamber, immediately following the introduction of sterilant vapor and carrier gas into the chamber, in response to the measured pressure.

### Summary of the Invention

A method and apparatus for vaporizing a liquid oxidizing agent per se or solution thereof comprises a tank containing the liquid, a vaporizer operatively connected to the tank, a flow control valve between the tank and the vaporizer, and wherein the vaporizer is capable of vaporizing the liquid oxidizing agent or solution. A vessel under a vacuum is operatively connected to the vaporizer and thus also applies a vacuum thereto. The vapor from the vaporizer is drawn into the vessel which contains a pressure sensor to detect the partial pressure of the vaporized oxidizing agent per se. A microprocessor is programmed to determine the pressure at which the vaporized oxidizing agent in the vessel would condense, as based upon the size of the vessel, and the partial pressure thereof. The microprocessor is connected to the vessel pressure sensor to parametrically control the flow control valve. Upon detection of a predetermined pressure, the flow control valve is closed thereby stopping the flow of liquid oxidizing agent or solution to the vaporizer and hence vaporized oxidizing agent to the vessel. Subsequently, a vacuum is applied to the vessel to substantially remove the oxidizing agent therein. Upon the completion of one or more cycles, articles contained within the vessel are sterilized as well as the interior walls, etc. of the vessel.

### BRIEF DESCRIPTION OF THE DRAWINGS

FIG. 1 is a flow diagram showing various components of the apparatus and processes of the present invention including a tank containing the liquid oxidizer or solution, a flow control valve located between the tank and a vaporizer which is connected to a vessel. A microprocessor receives signals from various items and performs various duties as set forth in the specification, and

FIG. 2 is a partial cross-section elevation view of a vaporizer apparatus according to the present invention.

### DETAILED DESCRIPTION OF THE INVENTION

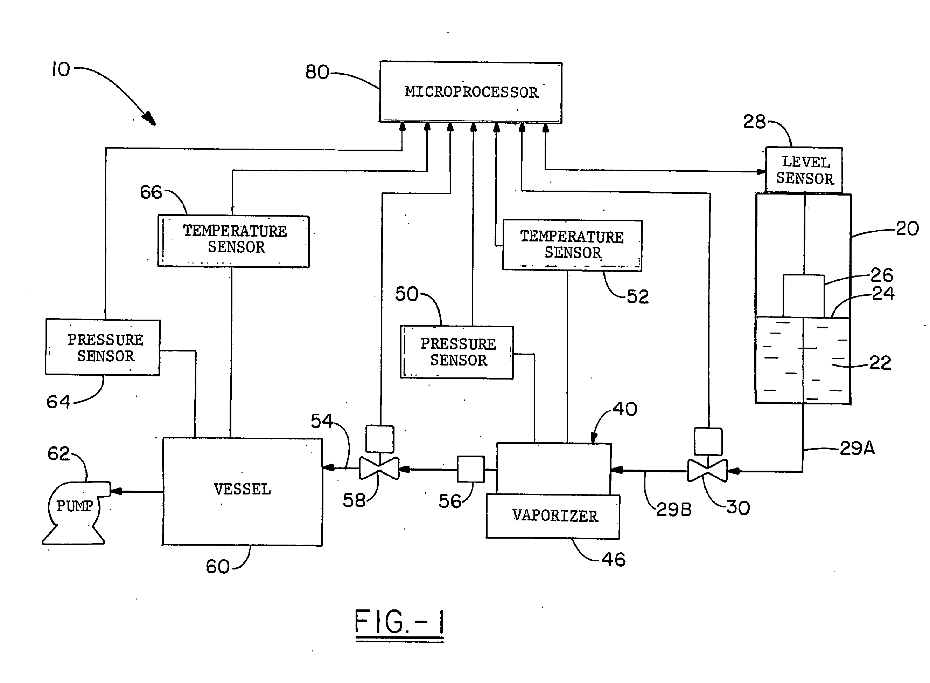

FIG. 1 relates to a flow diagram showing various components of the present invention such as tank 20, flow control value 30, vaporizer 40, vessel 60, and microprocessor 80.

Tank 20 contains a liquid oxidizing agent per se or solution 22 therein. The level of the liquid is indicated by numeral 24. Float 26 resides within tank 20 and is connected to a liquid oxidizing agent or solution sensor 28 which monitors the liquid level within the tank and hence the amount thereof. If the level is too low to conduct a sterilization cycle, microprocessor 60 detects a signal from sensor 28 with regard to a predetermined low level and prevents any vaporization of liquid 22 by closing control valve 30 until tank 20 is filled. Liquid 22 of tank 20 is fed via conduit 29A such as a pipe to flow control valve 30. The flow control valve is remotely controlled by microprocessor 80 such that it can be in a closed position, or an open position.

The oxidizing agents of the present invention include organic and inorganic peroxides, halogens, nitrates, and permanganates. Examples of peroxides include hydrogen peroxide, percarbonic acid, chlorine dioxide, permanganate, perlauric acid, perglutaric acid, or magnesium peroxyphthalate, and combinations thereof. Preferred compounds include peracetic acid and hydrogen peroxide. Halogens include chlorine, bromine, and iodine, various chlorates and perchlorates such as hypochlorites, various iodates and iodophors, and various bromates. Also included under the oxidizing agent listing of the present invention are various other biocides which include alcohols, aldehydes, and antimicrobial metals. The alcohols have from about 1 to about 6 carbon atoms and desirably include ethanol, n-propanol, and isopropanol. Examples of aldehydes include formaldehyde, glutaraldehyde, orthophthaldehyde, or formaldehyde-releasing agents such as hexamethylenetetramine, triazines, imidozoles, or hydantoins, and combinations thereof. Other agents which can be utilized include various alkylating agents such as ethylene oxide, propylene oxide, and the like. Such oxidizing agents are desirably in solution with a large amount of a suitable solvent such as water, although other solvents can be utilized such as alcohols having from 1 to about 6 carbon atoms. Generally the amount of the oxidizing agents such as hydrogen peroxide or peracetic acid are in concentrated form and can range from about 6% to about 70%, desirably from about 15% to about 50%, and preferably from about 35% to about 60% parts by weight based upon the total weight of the solution.

Vaporizer 40 can generally be a vessel of any shape, size or form in order to produce a desired amount of vaporized oxidizing agent or solution. Vaporizer 40 is generally small in size in comparison to vessel 60. Desirably the vaporizer has an internal volume of from about 0.25 to about 5 liters and desirably from about 0.50 to about 2 liters whereas vessel 60 can have an internal volume of from about 20 liters to about 40 cubic meters. Vaporizer 40 can be made of pharmaceutical grade materials such as stainless steel, aluminum, various fluoropolymers such as polytetrafluoroethylene, and the like. Vaporizer 40 has lid or cover 42 which forms an internal enclosure. The liquid oxidizing agent or solution is admitted to the vaporizer through nozzle 44. The nozzle can disperse the liquid oxidizing agent or solution generally in small or fine sized liquid particles or droplets, continuously, or in pulses. For example, a continuous nozzle can be utilized that manually or electronically controls the amount of oxidizing agent or solution dispersed into the vessel. Another suitable nozzle is an atomizer that disperses the oxidizing agent or solution as a very fine spray. Alternatively, the oxidizing agent or solution can be dispersed by a jet nozzle onto a heated surface. Still another suitable nozzle is a pulsating nozzle that disperses the oxidizing agent or solution in regulated pulses thereby permitting the liquid sufficient time to vaporize before the next pulse. Upon vaporization of the oxidizing agent or solution, the vaporizer, e.g. walls, floor, etc. are sterilized.

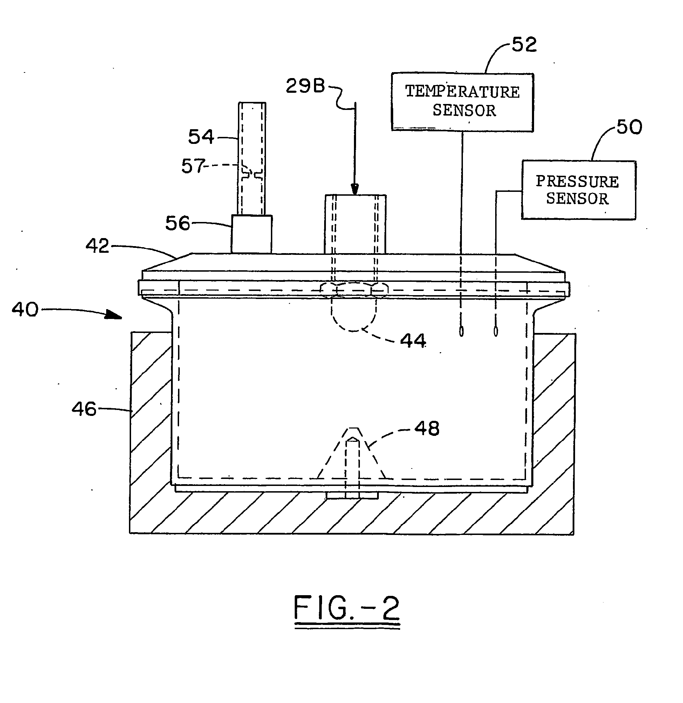

Another important aspect of the present invention is that the vaporizer is of a sufficient temperature to ensure rapid vaporization of the liquid oxidizing agent or solution. Accordingly, the vaporizer can have heated walls 46 which are heated in a conventional manner such as through the use of steam within the walls, electrical resistant heaters, and the like. Desirably, a heating element 48 is utilized and can be of any shape, size or surface area to permit rapid vaporization of the liquid oxidizing agent or liquid oxidizing solution. FIG. 2 shows a conical heating element which increases in surface area from an upper level to a lower level. Heating element 48 can be heated in any conventional manner such as through hot fluids, for example water; photonic radiation such as microwaves, infra red light, visible light, ultra violet light, X-rays, and so forth; sonic waves; electrical energy; and the like. To ensure complete vaporization, the heating element temperature as well as the temperature within the vaporizer should be above the boiling point of the oxidizing agent or solution thereof at the pressure existing within the vaporizer and the same will vary with the type and concentration of oxidizing agent. Suitable vaporization temperatures are generally from about 20°C to about 150°C and desirably from about 40°C to about 120°C. Such temperatures are the initial operating temperatures as well as generally the subsequent vaporizing temperatures since lower temperatures can result in condensation of the oxidizing agent.

An important aspect of vaporizer 40 is the existence of pressure sensor 50 which can be a pressure transducer. As shown in FIG. 1, pressure sensor 50 is connected to microprocessor 80. The temperature within the vaporizer is also monitored by temperature sensor 52 which also is connected to microprocessor 80. Vaporizer 40 during operation exists under a strong vacuum, that is at very low pressures which will be explained further herein below. After vaporization the liquid oxidizing agent or solution 22, the vapor is exhausted through vaporizer exhaust conduit 54. To prevent condensation of the vapor during transit, desirably exhaust conduit 54, that can be a pipe, is made out of a low heat conductive material such as plastic or the like. To further ensure that condensation does not occur, an optional external heating element 56 is contained generally in the vicinity of the exhaust portion of vaporizer 40. Heating element 56 can be an electric resistor heating element which heats the vapor to a higher temperature. An important component of exhaust conduit 54 is the optional but preferred existence of variable orifice 57. The orifice can have a large opening or small opening such as about 5% to about 95% of the total conduit opening to control the flow of the vaporized oxidizing agent or solution to the vessel and to prevent condensation thereof.

Vaporizer 40 can be specifically made for the present invention, or existing vessels can be economically converted such as freeze-drying vessels, washers, locks, transfer chambers, sterilization chambers, reaction vessels, and the like. Freeze-drying vessels relate to the freezing of items such as food with subsequent sublimation drying to remove water. The basic requirement of such vessels is that they are made of pharmaceutical grade materials as noted above with respect to the vaporizers so they are corrosion resistant to the oxidizing agent or solution and are able to withstand desired vacuum levels.

The vaporized oxidizing agent or solution is transferred via exhaust conduit 54 to vessel 60. As noted above, vessel 60 is much larger than vaporizer 40 and generally is utilized as a sterilizer to sterilize various articles placed therein. Also during sterilization the vessel itself, e.g. walls, floor, etc., is sterilized. An important aspect of the present invention is that vessel 60 contains a strong vacuum, that is a very low pressure. Vacuum pump 62 is utilized to withdraw a substantial amount of air, not only from vessel 60 but also from vaporizer 40 that is connected thereto by conduit 54. Conduit valve 58 is generally maintained in an open position. The initial vacuum within vessel 60, and hence vaporizer 40 is generally from about 0.01 millibar to about 0.5 millibar or optionally up to about 5, 10, 15, 20, 25, or about 30 millibars. As the vaporized oxidizing agent or solution is added thereto, naturally the pressure will increase. According to the present invention, pressure within vessel 60 is allowed to increase to a point below the condensation pressure of the oxidizing agent. Such pressures can readily be calculated based upon the volume of a vessel, the amount of vaporized oxidizing agent or solution added thereto, and also the temperature within the vessel. With regard to the preferred embodiment, often the pressure rises up to about 7 or to about 10 millibars or optionally up to about 20, 30, 40, or 50, or about 75 millibars.

The pressure within vessel 60 is determined by pressure sensor 64 which can be a pressure transducer. As shown in FIG. 1, the pressure transducer is electronically connected to microprocessor 80. Similarly, temperature sensor 66 is also electronically connected to microprocessor 80. The temperature within vessel 60 is generally less than that of vaporizer 40 and can range from about ambient, for example 10°C to about 40°C, and desirably from about 15°C to about 25°C, with about 20°C or about 21°C being preferred. Such low temperatures, if necessary, are generally maintained throughout the sterilizing cycles of the vessel. Heating and maintaining the vessel temperature can be achieved by heating the vessel walls, floors, etc. of the vessel in any manner as noted with regard to the vaporizer, for example use of hot fluids or steam within the walls, photonic radiation such as microwave, infra-red light, etc.

Vessel 60 can be a sterilizer per se or can be converted from existing vessel such as washers, freeze-dryers, locks, reaction vessels, transfer chambers, pressure vessels, and the like for use as a sterilizer.

Desirably the interior of the sterilization vessel is physically cleaned so as to remove dirt, scum, and the like before the vaporized oxidizing agent or solution is admitted thereto which naturally sterilizes the walls and surfaces of the sterilization vessel, including any absorbed materials. Cleaning of the vessel can occur before each sterilization cycle or after a predetermined number of cycles. Several different cleaning methods can be utilized such as the use of hot water which is either manually sprayed on all surfaces or automatically sprayed on the walls by the vessel having nozzles therein so that all surfaces are contacted.

The sterilization vessel can have any desired shape, size, or form, such as rectangular, so that it can contain articles therein that are to be disinfected and/or sterilized and accordingly can contain supports, platforms, tables, stands, hangers, etc. to hold, accommodate, etc., the articles to be disinfected or sterilized.

Microprocessor 80 can be programmed upon receipt of information through the various noted sensors and upon reaching a predetermined limit, to operate valve 30 by opening or closing the same. Moreover, when the level of the oxidizing agent or solution in tank 20 becomes deficient, the microprocessor can terminate the various vaporizing, sterilizing, etc., cycles.

The operation of the parametric apparatus sterilization system of the present invention is as follows. Generally vessel 60 has been previously cleaned in a manner as noted above. After cleaning, the vessel can either be sterilized by the vaporized oxidizing agent or solution as noted hereinabove or often, it is co-sterilized. That is, one or more articles are placed within the vessel to be sterilized and the vessel closed. Upon admitting the vaporized liquid oxidizing agent or solution, both the articles and the vessel walls, floor, ceiling, shelves, etc. will be sterilized. To aid in sterilization of the vessel, the vaporized liquid oxidizing agent or solution can be directed onto the walls of the vessel.

Once the articles have been added to vessel 60, a very strong vacuum is pulled by pump 62 so the vessel has a very low pressure therein, as previously noted such as from about 0.01 millibars to about 0.5 millibars preferred, or optionally up to about 5, 10, 15, 20, 25, or about 30 millibars. Since conduit 54 is generally open between vaporizer 40 and vessel 60, a vacuum is also applied to the vaporizer with substantially all the air therein being removed and the pressure therein being substantially the same as in vessel 60.

Once vacuums have been applied to vessel 60 and vaporizer 40, control valve 30 is opened and an amount of liquid oxidizing agent or solution 22 is added as controlled by microprocessor 80. The total amount of liquid to be added is that required to sterilize vessel 60 as well as the articles therein and the same is generally estimated by knowing the size of the vessel, the concentration of the oxidizing agent in liquid 22, the temperature in vessel 60, and the maximum amount of pressure tolerated within vessel 60 before condensation occurs. The same is thus calculated and programmed into the microprocessor. Thus for a given sterilization, an amount "X" of vaporized oxidizing agent or solution is required. That is, the total amount of the vapor therefrom contains a sufficient amount of oxidizing agent per se therein to achieve sterilization. While it is often desired that the total "X" amount of liquid 22 be added at one time to vaporizer 40 to generate the necessary volume of vapor, the same is generally not desired inasmuch as it is more efficient to vaporize the liquid in pulses. That is, a fractional amount of the required liquid 22 is added and vaporized so as not to overburden vaporizer 40 and cause condensation therein. The number and duration of pulses can thus be regulated and calculated by microprocessor 80. Should an excess amount of liquid 22 be added to vaporizer 40, an excess pressure buildup will be sensed by pressure sensor 50 which sends a signal to the microprocessor so that upon reaching or exceeding a pre-determined level, flow control valve 30 is closed. Pressure sensor 50 thus operates independently of pressure sensor 64.

As the vaporized oxidizing agent or solution gradually builds up in vessel 60, the pressure thereof is increased. Rather than to actually sample vessel 60 to determine the concentration of the oxidizing agent therein, the same is parametrically determined by the build up of the partial pressure of the vaporized oxidizing agent which is determined by pressure sensor 64. Upon reaching a predetermined level such as about 7 to about 10 millibars for a particular sterilization which was conducted, the pressure limit serves as a fairly accurate indicator of the amount of oxidizing agent in vessel 60. As noted above, condensation of the oxidizing agent per se is optimally avoided in vessel 60. This is because condensation on the surfaces of vessel 60, and the shelves thereof, and the like will eventually corrode the same. Condensation of the oxidizing agent also amounts to wasted agent since it is not utilized. Despite these disadvantages, condensation may be desired and similarly controlled under the same invention. Once the required pressure level in vessel 60 is reached, flow valve 30 is closed and the articles in vessel 60 are sterilized for a predetermined period of time. The sterilizer is then evacuated via a vacuum pump 62 to remove the oxidizing agent and any solution and the oxidizing agent such as hydrogen peroxide is treated with a catalyst to decompose the same to water and oxygen. If the sterilization cycle described immediately above did not utilize the calculated "X" amount of liquid 22, the cycle is repeated, (i.e. pulsed amounts of liquid 22 are vaporized and the same are fed to vessel 60 to sterilize the articles) until the required amount has been utilized and the articles are sterilized. A final vacuum is pulled to remove the oxidizing agent and the sterilized articles are then removed. After pressure is increased to atmospheric a new sterilizing cycle or plurality of sterilizing cycles can then be repeated with regard to other articles which require sterilization.

As noted above, vessel 60 can be freeze-dryer so that optionally during the sterilization cycle, the articles within vessel 60 if containing a fluid therein, can be frozen and a vacuum applied to sublimate the same and yield a solid dried article.

Articles that can be placed in the sterilization vessel and treated according to the present invention are known to the art and to the literature and include numerous types of medical and surgical instruments, various types of equipment which require disinfection or sterilization such as small pharmaceutical plastic parts of any kind and form (e.g. plastic caps, stoppers); various food stuffs e.g. packages; and the like. Moreover, other vacuum based systems can serve as sterilization vessels that require routine decontamination and include electron microscopes and other microbiological investigation equipment. Other articles include vials which contain liquid pharmaceutical compounds therein wherein the solvent, solution, or water is to be removed and the remaining solvent sterilized.

The vaporized oxidizing agents or solutions according to the present invention can inactivate, disinfect or sterilize a large number of microorganisms such as bacteria, fungi, viruses, prions, as well as to detoxify various toxins produced by microorganisms and various drugs, including cytotoxic drugs. Examples of microorganisms include endospores such as *Geobacillus stearothermophilus, Bacillus subtilis, Bacillus subtilis globigii, Clostridium sporogenes, Bacillus cereus,* or *Bacillus circulans* or combinations thereof; various fungi such as *Aspergillus niger, Candida albicans, Trichophyton mentagrophytes,* or *Wagiella dermatitis,* or combinations thereof; various mycobacteria such as *Mycobacterium chelonae, Mycobacterium gordonae, Mycobacterium smegmantis,* or *Mycobacterium terrae,* or combinations thereof; various vegetative bacteria such as *Aeromonas hydrophila, Enterococcus faecalis, Streptococcus faecalis, Enterococcus facecium, Streptococcus pyrogenes, Escherichia coli, Klebsiella (pneumoniae), Legionella pneumophila, Methylobacterium, Pseudomonas aeruginosa, Salmonella chloreraesuis, Helicobacter pylori, Staphylococcus aureus, Staphylococcus epidermidis,* or *Stenotrophomonas maltophilia,* or combinations thereof; and various protozoa such as *Giardia lamblia,* or *Cryptosporidium parvum,* or combinations thereof. Examples of infectious proteins include the prions identified as PrP^{Sc}. Drugs which can be detoxified or chemically reacted by the present invention include various drugs that are sensitive to the effects of oxidizing agents including cytotoxic drugs such as 5-fluorouracil, cyclophosphamide and doxorubicin. Other undesirable contaminants can also be degraded including nucleic acids, proteins, lipids and carbohydrates.

The invention will be better understood by the following examples which serve to illustrate, but not to limit the same.

A vaporizer was charged with hydrogen peroxide solution in accordance with the following table. Two separate runs were made.

**Table 1**

| **Vaporizer** | **Example A** | **Example B** |
|---|---|---|
| Initial vacuum in vaporizer | 0.01 mbar | 0.5 mbar |
| Temperature range vaporizer | 130 to 150°C | 60 to 120°C |
| Temperature heating element | 150°C | 120°C |
| H₂O₂ solution | 35 m-% | 35 m-% |
| Injected solution - 2 pulses | 20.0 ml/15.2 ml | 12.4 ml/ 9.9 ml |

The vapor generated by the vaporizer was then fed to a vessel which was a freeze-dryer. With regard to Example A, the vessel volume portion of the freeze-dryer was 2,400 ml. The initial pressure was 0.01 mbar and the vessel temperature was approximately 21 °C. The two injection solution pulses were drawn into the vessel. The hold time after each injection was about fourteen minutes. The pressure range within the vessel after admitting the vaporized liquid was about 7 mbar. Articles within the vessel were located on shelves. After the sterilization cycle was completed, Drager test analysis revealed that the amount of residual biocide within the articles (i.e. vials containing pharmaceutical compounds) was only 2 to 3 parts per million.

While in accordance with the patent statutes, the best mode and preferred embodiment have been set forth, the scope of the invention is not limited thereto, but rather by the scope of the attached claims.

## Claims

1. An apparatus for vaporization of an oxidizing agent or solution and sterilization of at least one article, comprising:
a tank (20) containing a liquid oxidizing agent or solution (22); said tank having a float (26) therein connected to a liquid level sensor (28) for determining the level of said oxidizing agent or solution in said tank;
a vaporizer (40), said vaporizer operatively connected to said tank by a conduit (29A), said conduit (29A) having a flow control valve (30) for admitting said oxidizing agent or solution to said vaporizer (40), said vaporizer having an injection nozzle (44) for admitting said oxidizing agent or solution to said vaporizer, said vaporizer (40) having a heating element (48) for heating said vaporizer and a pressure sensor (50) for sensing the pressure inside said vaporizer, said vaporizer (40) being capable of having a vacuum applied thereto and being capable of vaporizing said oxidizing agent or solution (22) admitted thereto, said vaporizer having an exhaust conduit (54) having a conduit valve (58) therein which is generally maintained in an open position;
a vessel (60), said exhaust conduit (54) operatively connected to said vessel, said vessel having a pressure sensor (64) therein for determining the partial pressure of said vaporized oxidizing agent inside said vessel, said vessel capable of receiving at least one article therein to be sterilized;
a vacuum pump (62) capable of applying a vacuum to said vessel (60) and to said vaporizer (40), said vessel operatively connected to said vacuum pump (62); and
a microprocessor (80), said microprocessor (80) being capable of receiving a signal from said vessel pressure sensor (64), from said vaporizer pressure sensor (50) and from said liquid level sensor (28), said microprocessor (80) being connected to said control valve (30) and capable of operating said control valve (30) by opening or closing the same such that said liquid oxidizing agent or solution (22) is vaporized in pulses, wherein
said microprocessor is programmed and capable of parametrically determining the amount of said oxidizing agent in said vessel based upon said pressure signal and to close said control valve upon said pressure sensor detecting a predetermined pressure in said vessel to prevent or ensure condensation of said oxidizing agent in said vessel,
wherein said vaporizer pressure sensor (50) is capable of sending a signal to said microprocessor (80), and wherein said microprocessor upon detecting a predetermined pressure level in said vaporizer (40) is capable of closing said flow control valve (30), and
wherein said liquid tank level sensor (28) is capable of sending a signal to said microprocessor (80), and wherein said microprocessor upon detecting a predetermined low level is capable of closing said flow control valve (30).

2. The apparatus of claim 1, further including a variable orifice in said exhaust conduit.

3. The apparatus of claim 2, wherein said oxidizing agent or solution comprises an organic or inorganic peroxide, a halogen, a nitrate, a permanganate, an alcohol, an aldehyde, an alkylating agent, or an antimicrobial metal, or combinations thereof, and optionally including a heater on said exhaust conduit.

4. The apparatus of claim 3, wherein said vessel is a washer, a freeze-dryer, a reaction vessel, a lock, a transfer chamber, a pressure vessel, or a sterilizer; including said variable exhaust orifice, and wherein said orifice opening is from about 5 % to about 95 % of said exhaust conduit opening.

5. The apparatus of claim 4, wherein said vessel is said freeze-dryer, wherein said oxidizing agent is hydrogen peroxide, peracetic acid, ozone or chlorine dioxide, or alternative biocides including glutaraldehyde, orthophthaldehyde, or formaldehyde, or said alcohol, or combinations thereof; and including said exhaust conduit heater, and wherein said apparatus is capable of sterilizing said article, or said vessel, or both.

6. A method for the vaporization of an oxidizing agent or solution and sterilization of at least one article, or a vessel, or both, comprising the steps of:
providing a tank (20) containing a liquid oxidizing agent or solution (22); said tank having a float (26) therein connected to a liquid level sensor (28);
providing a vaporizer (40) capable of having a vacuum applied thereto, admitting only said liquid oxidizing agent or solution into said vaporizer through an injection nozzle (44), said vaporizer having a heating element (48) for heating said vaporizer and a pressure sensor (50) for sensing the pressure inside said vaporizer, said vaporizer operatively connected to said tank by a conduit (29A) having a flow control valve (30) therein;
providing a vacuum pump (62);
providing a vessel (60), said vessel operatively connected to said vacuum pump, said vaporizer (40) operatively connected to said vessel by a vaporizer exhaust conduit (52) having a conduit valve (58) therein, said vessel (60) having a pressure sensor (64) for determining the partial pressure of said oxidizing agent inside said vessel;
providing a microprocessor (80) connected to said vessel pressure sensor (64), to said vaporizer pressure sensor (50), to said liquid level sensor (28) and to said control valve (30);
programming said microprocessor (80) upon receipt of information through the liquid level sensor (28), the vaporizer pressure sensor (50) and the vessel pressure sensor (64), wherein the total amount of liquid oxidizing agent to be admitted into said vaporizer is calculated and programmed into said microprocessor such that if a sterilization cycle did not utilize the calculated total amount of liquid oxidizing agent the cycle is repeated;
applying an initial vacuum of about 0.01 to about 30 millibars to said vessel (60) with said vacuum pump (62) and to said operatively connected vaporizer (46) and substantially removing air therefrom so that the pressure inside said vessel and the pressure inside said vaporizer are lower than the pressure in said tank (20);
maintaining said conduit valve (58) generally in an open position such that the initial vacuum pressure in said vessel (60) and in said vaporizer (40) are the same;
admitting only said oxidizing agent or said solution (22) to said vaporizer (40) through said control valve (30) and vaporizing said liquid oxidizing agent or solution in said vaporizer, wherein said microprocessor (80) operates said control valve (30) by opening or closing the same such that said liquid oxidizing agent or solution (22) is vaporized in pulses;
said vaporizer pressure sensor (50) sending a signal to said microprocessor (80) upon sensing an excess pressure buildup when an excess amount of liquid oxidizing agent is added to said vaporizer (40), wherein said microprocessor closes said control valve (30) upon reaching or exceeding a predetermined level of liquid oxidizing agent in said vaporizer;
admitting said vaporized oxidizing agent or solution (22) from said vaporizer (40) to said vessel (60);
said liquid level sensor (28) continuously monitoring and determining the level of said oxidizing agent or solution (22) in said tank (20), wherein said microprocessor (80) detects a signal from said liquid level sensor with regard to a predetermined low level and closes said control valve (30) in order to prevent any vaporization of liquid until said tank is filled;
said microprocessor (80) closing said control valve (30) upon detecting a predetermined partial vapor pressure of said vaporized oxidizing agent in said vessel (40).

7. The method of claim 6, wherein said vaporizer exhaust conduit (54) contains a variable orifice.

8. The method of claim 7, wherein said oxidizing agent or solution (22) comprises an organic or inorganic peroxide, a halogen, a nitrate, a permanganate, an alcohol, an aldehyde, an alkylating agent, or an antimicrobial metal, or combinations thereof, and optionally including a heater on said exhaust conduit.

9. The method of claim 8, wherein said vessel (60) comprises a washer, a freeze-dryer, a reaction vessel, a lock, a transfer chamber, a pressure vessel, or a sterilizer; wherein the temperature in said vessel is from about 10°C to about 40°C, and including a variable exhaust orifice, wherein said variable exhaust orifice has an opening of from about 5 % to about 95 % of said exhaust conduit opening, and wherein said initial vessel vacuum level is from about 0.01 millibar to about 15 millibar.

10. A method of claim 9, wherein said vessel (60) is said freeze-dryer, wherein the vaporization temperature is from about 20° C to about 150° C, and wherein said predetermined partial vapor pressure of said vaporized oxidizing agent in said vessel is from about 7 millibar to about 50 millibar.

11. The method of claim 10, wherein said oxidizing agent is hydrogen peroxide, peracetic acid, or chlorine dioxide, or alternative biocides including glutaraldehyde, orthophthaldehyde, or formaldehyde, or said alcohol, or combinations thereof; wherein said initial vacuum pressure is from about 0.01 millibar to about 0.5 millibar including an exhaust conduit heater, admitting said liquid oxidizing agent or solution to said vaporizer (40) by a plurality of pulses through said injection nozzle (44) and wherein said vessel temperature is about 15° C to about 25° C.

## Patentansprüche

1. Vorrichtung zum Verdampfen eines Oxidationsmittels oder einer Oxidationsmittellösung zur Sterilisation zumindest eines Gegenstands, mit:
einem Vorratsbehälter (20), der ein flüssiges Oxidationsmittel oder eine Oxidationsmittellösung (22) enthält; wobei der Vorratsbehälter einen Schwimmer (26) aufweist, welcher mit einem Flüssigkeitspegelsensor (28) verbunden ist, um den Pegel des Oxidationsmittels oder die Oxidationsmittellösung in dem Vorratsbehälter zu bestimmen;
einem Verdampfer (40), der betriebsmäßig über eine Rohrleitung (29A) mit dem Vorratsbehälter verbunden ist, wobei sich in der Rohrleitung (29A) ein Durchflussregelventil (30) befindet, das den Zufluss des Oxidationsmittels oder der Oxidationsmittellösung zu dem Verdampfer (40) regelt, wobei sich in dem Verdampfer eine Einlassdüse (44) zum Einlassen des Oxidationsmittels oder der Oxidationsmittellösung in den Verdampfer befindet, wobei der Verdampfer (40) mit einem Heizelement (48) zur Erwärmung des Verdampfers und mit einem Drucksensor (50) zum Erfassen des Drucks in dem Verdampfer ausgerüstet ist, wobei ein Unterdruck an den Verdampfer (40) angelegt werden kann und der Verdampfer im Stande ist, das zugeführte Oxidationsmittel oder die Oxidationsmittellösung (22) zu verdampfen, wobei der Verdampfer mit einer Abgasleitung (54) ausgerüstet ist, in welcher ein Rohrleitungsventil (58) vorgesehen ist, das im Allgemeinen in einer offenen Position gehalten ist;
einem Gefäß (60), das betriebsmäßig mit der Abgasleitung (54) verbunden ist, wobei sich in dem Gefäß ein Drucksensor (64) zur Bestimmung des Partialdrucks des verdampften Oxidationsmittels befindet, wobei das Gefäß im Stande ist, zumindest einen zu sterilisierenden Gegenstand aufzunehmen;
einer Vakuumpumpe (62), die im Stande ist, einen Unterdruck in dem Gefäß (60) und dem Verdampfer (40) zu erzeugen, wobei das Gefäß betriebsmäßig mit der Vakuumpumpe (62) verbunden ist; und
einem Mikroprozessor (80), wobei der Mikroprozessor (80) im Stande ist, ein Signal von dem Gefäß-Drucksensor (64), von dem Verdampfer-Drucksensor (50) und von dem Flüssigkeitspegelsensor (28) zu empfangen, wobei der Mikroprozessor (80) mit dem Regelventil (30) verbunden und in der Lage ist, das Regelventil (30) zu betätigen, indem es dieses derart öffnet oder schließt, dass das Oxidationsmittel oder die Oxidationsmittellösung (22) in Impulsen verdampft wird,
wobei der Mikroprozessor so programmiert ist und dazu im Stande ist, die Menge des Oxidationsmittels in dem Gefäß parametrisch aus dem Drucksignal zu berechnen und, wenn der Drucksensor einen vorbestimmten Druck im Gefäß festgestellt hat, das Durchflussregelventil zu schließen, um eine Kondensation des Oxidationsmittels in dem Gefäß zu verhindern oder zu gewährleisten,
wobei der Verdampfer-Drucksensor (50) in der Lage ist, ein Signal an den Mikroprozessor (80) zu senden, und wobei der Mikroprozessor in der Lage ist, bei Erkennen eines vorbestimmten Druckpegels in dem Verdampfer (40), das Durchflussregelventil (30) zu schließen, und
wobei der Flüssigkeitsvorratsbehälter-Pegelsensor (28) in der Lage ist, ein Signal an den Mikroprozessor (80) zu senden, und wobei der Mikroprozessor in der Lage ist, bei Erkennen eines vorbestimmten niedrigen Pegels das Durchflussregelventil (30) zu schließen.

2. Vorrichtung gemäß Anspruch 1, ferner mit einer variablen Öffnungsblende in der Abgasleitung.

3. Vorrichtung gemäß Anspruch 2, bei welcher das Oxidationsmittel oder die Oxidationsmittellösung ein organisches oder anorganisches Peroxyd, ein Halogen, ein Nitrat, ein Permanganat, einen Alkohol, ein Aldehyd, Alkylanzien, oder ein antimikrobiell wirkendes Metall, oder deren Kombinationen enthält, und die Abgasleitung wahlweise mit einer Heizung ausgerüstet ist.

4. Vorrichtung gemäß Anspruch 3, bei welcher das Gefäß ein Waschbehälter, ein Gefriertrocknungsbehälter, eine Reaktionskammer, eine Schleusenkammer, eine Übergabekammer, ein Druckbehälter oder ein Sterilisator ist, welche jeweils die variable Öffnungsblende aufweisen, und wobei die Öffnung der Öffnungsblende einen Bereich von etwa 5% bis etwa 95% der Öffnung der besagten Abgasleitung abdeckt.

5. Vorrichtung gemäß Anspruch 4, bei welcher das Gefäß ein Gefriertrockner ist, wobei das Oxidationsmittel Wasserstoffperoxid, Peressigsäure, Ozon oder Chlordioxid, oder andere Biozide, beispielsweise, Glutaraldehyd, Orthophthaldehyd, Formaldehyd oder Alkohol ist, oder aus Kombinationen dieser Stoffe besteht, und wobei die Vorrichtung die Abgasleitungsheizung aufweist, und wobei die Vorrichtung im Stande ist, den Gegenstand, das Gefäß oder beides zu sterilisieren.

6. Verfahren zum Verdampfen eines Oxidationsmittels oder einer Oxidationsmittellösung und zur Sterilisation zumindest eines Gegenstand, oder eines Gefäßes oder von beidem, das folgende Schritte umfasst:
das Bereitstellen eines Vorratsbehälters (20), der ein flüssiges Oxidationsmittel oder eine Oxidationsmittellösung (22) enthält, wobei der Vorratsbehälter einen Schwimmer (26) aufweist, welcher mit einem Flüssigkeitspegelsensor (28) verbunden ist;
das Bereitstellen eines Verdampfers (40), in dem ein Unterdruck erzeugt werden kann, wobei über eine Injektionsdüse (44) nur das flüssige Oxidationsmittel oder die Oxidationsmittellösung in den Verdampfer eingeleitet wird, wobei der Verdampfer mit einem Heizelement (48) zum Aufheizen des Verdampfers und mit einem Drucksensor (50) zum Erkennen des Drucks in dem Verdampfer versehen ist, wobei der Verdampfer betriebsmäßig mit dem Vorratsbehälter über eine Rohrleitung (29A) verbunden ist, in welcher ein Durchflussregelventil (30) vorgesehen ist;
das Bereitstellen einer Vakuumpumpe (62);
das Bereitstellen eines Gefäßes (60), wobei das Gefäß betriebsmäßig mit der Vakuumpumpe verbunden ist, wobei der Verdampfer (40) über eine mit einem Leitungsventil (58) versehene Verdampfer-Abgasleitung (52) betriebsmäßig mit dem Gefäß verbunden ist, wobei das Gefäß (60) mit einem Drucksensor (64) zum Feststellen des Partialdrucks des Oxidationsmittels in dem Gefäß ausgerüstet ist;
das Bereitstellen eines mit dem Gefäß-Drucksensor (64), dem Verdampfer-Drucksensor (50), dem Flüssigkeitspegelsensor (28) und dem Regelventil (30) verbundenen Mikroprozessors (80);
das Programmieren des Mikroprozessors (80) bei Empfang von Informationen über den Flüssigkeitspegelsensor (28), den Verdampfer-Drucksensor (50) und den Gefäß-Drucksensor (64), wobei die Gesamtmenge des in den Verdampfer einzuleitenden flüssigen Oxidationsmittels berechnet und derart in den Mikroprozessor programmiert wird, dass, falls ein Sterilisationszyklus nicht die berechnete Gesamtmenge des flüssigen Oxidationsmittels verwendet hat, der Zyklus wiederholt wird;
das Erzeugen eines anfänglichen Unterdrucks von etwa 0,01 bis etwa 30 Millibar mittels der Vakuumpumpe (62) in dem Gefäß (60) und dem betriebsmäßig damit verbundenen Verdampfer (46), und das wesentliche Entfernen von Luft aus diesem, so dass der Druck in dem Gefäß und der Druck in dem Verdampfer niedriger als der Druck in dem Vorratsbehälter (20) sind;
das Halten des Rohrleitungsventils (58) im Wesentlichen in einer offenen Position, derart, dass der anfängliche Unterdruck in dem Gefäß (60) und in dem Verdampfer (40) gleich sind;
das Einleiten nur des Oxidationsmittels oder der Oxidationsmittellösung (22) durch das Durchflussregelventil (30) in den Verdampfer (40) und das Verdampfen des flüssigen Oxidationsmittels oder der Oxidationsmittellösung in dem Verdampfer, wobei der Mikroprozessor (80) das Regelventil (30) durch Öffnen und Schließen desselben betätigt, derart, dass das flüssige Oxidationsmittel oder die Oxidationsmittellösung (22) in Impulsen verdampft wird;
wobei der Verdampfer-Drucksensor (50) ein Signal an den Mikroprozessor (80) sendet, wenn er einen übermäßigen Druckaufbau erkennt, der bei Zufuhr einer Übermenge an flüssigem Oxidationsmittel in den Verdampfer (40) entsteht, wobei der Mikroprozessor das Regelventil (30) schließt, wenn in dem Verdampfer ein vorbestimmter Pegel des flüssigen Oxidationsmittels erreicht oder überschritten wird;
das Einleiten des verdampften Oxidationsmittels oder der verdampften Oxidationsmittellösung (22) aus dem Verdampfer (40) in das Gefäß (60);
wobei der Flüssigkeitspegelsensor (28) den Pegel des Oxidationsmittels oder die Oxidationsmittellösung (22) in dem Vorratsbehälter (20) kontinuierlich überwacht und feststellt, wobei der Mikroprozessor (80) ein Signal des Flüssigkeitspegelsensors hinsichtlich eines vorbestimmten niedrigen Pegels erkennt und das Regelventil (30) schließt, um jedes Verdampfen von Flüssigkeit zu verhindern, bis der Vorratsbehälter gefüllt ist;
wobei der Mikroprozessor (80) das Regelventil (30) schließt, wenn er einen vorbestimmten Dampfpartialdruck des verdampften Oxidationsmittels in dem Gefäß (60) erkennt.

7. Verfahren gemäß Anspruch 6, bei welchem sich in der Abgasleitung (54) eine variable Öffnungsblende befindet.

8. Verfahren gemäß Anspruch 7, bei welchem das Oxidationsmittel oder die Oxidationsmittellösung (22) ein organisches oder anorganisches Peroxyd, ein Halogen, ein Nitrat, ein Permanganat, einen Alkohol, ein Aldehyd, Alkylanzien, oder ein antimikrobiell wirkendes Metall, oder deren Kombinationen aufweist, und die Abgasleitung wahlweise mit einer Heizung ausgerüstet ist.

9. Verfahren gemäß Anspruch 8, bei welchem das Gefäß (60) einen Waschbehälter, einen Gefriertrockner, eine Reaktionskammer, eine Schleusenkammer, eine Übergabekammer, einen Druckbehälter oder einen Sterilisator aufweist, wobei die Temperatur in dem Gefäß in dem Bereich von etwa 10° C bis etwa 40°C liegt, und mit einer variablen Abgas-Öffnungsblende, wobei die Öffnung der variable Abgas-Öffnungsblende einen Bereich von etwa 5% bis etwa 95% der Öffnung der Abgasleitung abdeckt, und wobei der anfänglich in dem Gefäß herrschende Unterdruck im Bereich von etwa 0,01 Millibar bis etwa 15 Millibar liegt.

10. Verfahren gemäß Anspruch 9, bei welchem das Gefäß (60) ein Gefriertrockner ist, wobei die Verdampfungstemperatur zwischen ungefähr 20°C und ungefähr 150°C beträgt, und wobei der festgelegte Dampfpartialdruck des verdampften Oxidationsmittels in dem Gefäß in einem Bereich von etwa 7 Millibar bis etwa 50 Millibar liegt.

11. Verfahren gemäß Anspruch 10, bei welchem das Oxidationsmittel Wasserstoffperoxid, Peressigsäure, Chlordioxid, oder ein alternatives Biozid, beispielsweise Glutaraldehyd, Orthophthaldehyd, Formaldehyd oder Alkohol aufweist oder aus Kombinationen dieser Stoffe besteht, wobei der anfängliche Unterdruck zwischen ungefähr 0,01 Millibar und ungefähr 0,5 Millibar beträgt, wobei eine Abgasleitungsheizung vorgesehen ist, wobei das flüssige Oxidationsmittel oder die Oxidationsmittellösung in mehreren Pulsen durch das Durchflussregelventil durch die Injektionsdüse (44) in den Verdampfer (40) eingeleitet wird, und wobei die Temperatur in dem Gefäß im Bereich von etwa 15°C bis etwa 25°C liegt.

## Revendications

1. Appareil destiné à la vaporisation d'un agent oxydant liquide ou d'une solution oxydante et à la stérilisation d'au moins un article, comprenant :
un réservoir (20) contenant un agent oxydant liquide ou une solution oxydante (22) ; ledit réservoir comportant intérieurement un flotteur (26) relié à un détecteur de niveau de liquide (28) servant à déterminer le niveau dudit agent oxydant ou de ladite solution oxydante dans ledit réservoir ;
un vaporiseur (40), ledit vaporiseur étant relié fonctionnellement audit réservoir par un conduit (29A), ledit conduit (29A) ayant une vanne de commande d'écoulement (30) servant à admettre ledit agent oxydant ou ladite solution oxydante dans ledit vaporiseur (40), ledit vaporiseur ayant une buse d'injection (44) destinée à admettre ledit agent oxydant ou ladite solution oxydante dans ledit vaporiseur, ledit vaporiseur (40) comportant un élément chauffant (48) servant à chauffer le vaporiseur et un détecteur de pression (50) servant à détecter la pression dans ledit vaporiseur, ledit vaporiseur (40) pouvant recevoir l'application d'un vide et pouvant vaporiser ledit agent oxydant ou ladite solution oxydante (22) qui y est admis, ledit vaporiseur comprenant un conduit d'évacuation (54) qui présente intérieurement une vanne de conduit (58) qui est généralement maintenue dans une position ouverte ;
un récipient (60), ledit conduit d'évacuation (54) étant relié fonctionnellement audit récipient, ledit récipient présentant intérieurement un détecteur de pression (64) pour déterminer la pression partielle dudit agent oxydant vaporisé dans ledit récipient, ledit récipient pouvant recevoir intérieurement au moins un article à stériliser ;
une pompe à vide (62) capable d'appliquer un vide audit récipient (60) et audit vaporiseur (40), ledit récipient étant relié fonctionnellement à ladite pompe à vide (62) ; et
un microprocesseur (80), ledit microprocesseur (80) pouvant recevoir un signal en provenance dudit détecteur de pression du récipient (64), dudit détecteur de pression du vaporiseur (50), et dudit détecteur de niveau de liquide (28), ledit microprocesseur (80) étant relié à ladite vanne de commande (30) et pouvant actionner ladite vanne de commande (30) en ouvrant ou en fermant cette vanne de telle manière que ledit agent oxydant liquide ou ladite solution oxydante (22) soit vaporisé par impulsions,
ledit microprocesseur étant programmé pour déterminer et pouvant déterminer paramétriquement la quantité dudit agent oxydant présente dans ledit récipient sur la base dudit signal de pression et fermer ladite vanne de commande lorsque ledit détecteur de pression détecte une pression prédéterminée dans ledit récipient pour empêcher ou assurer la condensation dudit agent oxydant dans ledit récipient,
ledit détecteur de pression du vaporiseur (50) pouvant envoyer un signal audit microprocesseur (80) et ledit microprocesseur pouvant fermer ladite vanne de commande d'écoulement (30) en réponse à la détection d'un niveau de pression prédéterminé dans ledit vaporiseur (40) ; et
ledit détecteur de niveau de liquide du récipient (28) pouvant envoyer un signal audit microprocesseur (80), et ledit microprocesseur pouvant fermer ladite vanne de commande d'écoulement (30) en réponse à la détection d'un bas niveau prédéterminé.

2. Appareil selon la revendication 1, comprenant en outre un orifice variable dans ledit conduit d'évacuation.

3. Appareil selon la revendication 2, dans lequel ledit agent oxydant ou ladite solution oxydante comprend un peroxyde organique ou inorganique, un halogène, un nitrate, un permanganate, un alcool, un aldéhyde, un agent alkylant, ou un métal antimicrobien, ou des combinaisons de ces substances, et comprenant facultativement un élément chauffant sur ledit conduit d'évacuation.

4. Appareil selon la revendication 3, dans lequel ledit récipient est un appareil de lavage, un lyophilisateur, un récipient de réaction, un espace clos, une chambre de transfert, un récipient à pression ou un stérilisateur, qui comprend ledit orifice d'évacuation variable, et dans lequel l'ouverture dudit orifice est d'environ 5 % à environ 95 % de l'ouverture dudit conduit d'évacuation.

5. Appareil selon la revendication 4, dans lequel ledit récipient est ledit lyophilisateur, ledit agent oxydant étant le peroxyde d'hydrogène, l'acide acétique, l'ozone ou le dioxyde de chlore, ou d'autre biocides qui comprennent le glutaraldéhyde, l'orthophthaldéhyde ou le formaldéhyde, ou ledit alcool ou des combinaisons de ces substances ; et comprenant ledit élément chauffant du conduit d'évacuation, et ledit appareil étant capable de stériliser ledit article ou ledit récipient ou les deux.

6. Procédé de vaporisation d'un agent oxydant ou d'une solution oxydante et de stérilisation d'au moins un article, ou d'un récipient ou des deux, comprenant les étapes consistant à :
fournir un réservoir (20) qui contient un agent oxydant liquide ou une solution oxydante (22) ; ledit réservoir présentant intérieurement un flotteur (26) relié à un détecteur de niveau de liquide (28) ;
fournir un vaporiseur (40) pouvant recevoir l'application d'un vide, admettre exclusivement ledit agent oxydant liquide ou ladite solution oxydante dans ledit vaporiseur à travers une buse d'injection (44), ledit vaporiseur possédant un élément chauffant (48) servant à chauffer ledit vaporiseur et un détecteur de pression (50) servant à détecter la pression dans ledit vaporiseur, ledit vaporiseur étant relié fonctionnellement audit réservoir par un conduit (29A) ayant intérieurement une vanne de commande d'écoulement (30);
fournir une pompe à vide (62) ;
fournir un récipient (60), ledit récipient étant relié fonctionnellement à ladite pompe à vide, ledit vaporiseur (40) étant relié fonctionnellement audit récipient par un conduit d'évacuation du vaporiseur (52) qui a intérieurement une vanne de conduit (58), ledit récipient (60) comportant un détecteur de pression (64) servant à déterminer la pression partielle dudit agent oxydant dans ledit récipient ;
fournir un microprocesseur (80) relié audit détecteur de pression du récipient (64),
audit détecteur de pression du vaporiseur (50), audit détecteur de niveau du liquide (28) et à ladite vanne de commande (30) ;
programmer ledit microprocesseur (80) pour qu'à la réception d'une information par l'intermédiaire du détecteur de niveau de liquide (28), du détecteur de pression du vaporiseur (50) et du détecteur de pression du récipient (64), la quantité totale d'agent oxydant liquide qui doit être admise dans ledit vaporiseur soit calculée et programmée dans le microprocesseur de telle manière que si un cycle de stérilisation n'a pas utilisé la quantité totale calculée d'agent oxydant liquide, le cycle soit répété ;
appliquer un vide initial d'environ 0,01 à environ 30 millibars audit récipient (60), au moyen de ladite pompe à vide (62), et audit vaporiseur (46) qui y est relié fonctionnellement et en évacuer sensiblement l'air de telle manière que la pression dans ledit récipient et la pression dans ledit vaporiseur soient inférieures à la pression intérieure dudit réservoir (20) ;
maintenir ladite vanne du conduit (58) de façon générale dans une position ouverte de telle manière que la pression de vide initiale soit la même dans ledit récipient (60) et dans ledit vaporiseur (40) ;
admettre exclusivement ledit agent oxydant ou ladite solution oxydante (22) dans ledit vaporiseur (40) à travers ladite vanne de commande (30) et vaporiser ledit agent oxydant liquide ou ladite solution oxydante dans ledit vaporiseur, ledit microprocesseur (80) commandant ladite vanne de commande (30) en ouvrant ou
fermant cette vanne de telle manière que ledit agent oxydant liquide ou ladite solution oxydante (22) soit vaporisé(e) par impulsions ;
ledit détecteur de pression du vaporiseur (50) envoyant un signal audit microprocesseur (80) en réponse à la détection d'une accumulation de pression excessive lorsqu'une quantité excessive dudit agent oxydant liquide est ajoutée audit vaporiseur (40), ledit microprocesseur fermant ladite vanne de commande (30) en réponse à l'atteinte ou au dépassement d'un niveau prédéterminé d'agent oxydant liquide dans ledit vaporiseur ;
admettre ledit agent oxydant ou ladite solution oxydante vaporisé(e) (22) dudit vaporiseur (40) dans ledit récipient (60) ;
ledit détecteur de niveau de liquide (28) surveillant et déterminant continuellement le niveau dudit agent oxydant ou de ladite solution oxydante (22) dans ledit réservoir (20), ledit microprocesseur (80) détectant un signal issu du détecteur de niveau de liquide relativement à un bas niveau prédéterminé et fermant ladite vanne de commande (30) pour interdire la vaporisation de liquide jusqu'à ce que ledit réservoir soit rempli ;
ledit microprocesseur (80) fermant ladite vanne de commande (30) lorsqu'il détecte une pression partielle de vapeur prédéterminée dudit agent oxydant vaporisé dans ledit récipient (60).

7. Procédé selon la revendication 6, dans lequel ledit conduit d'évacuation du vaporiseur (54) présente un orifice variable.

8. Procédé selon la revendication 7, dans lequel ledit agent oxydant ou ladite solution oxydante (22) comprend un peroxyde organique ou inorganique, un halogène, un nitrate, un permanganate, un alcool, un aldéhyde, un agent alkylant ou un métal antimicrobien, ou des combinaisons de ces substances, et présentant facultativement un élément chauffant sur ledit conduit d'évacuation.

9. Procédé selon la revendication 8, dans lequel le dit récipient (60) comprend un appareil de lavage, un lyophilisateur, un récipient de réaction, un espace clos, une chambre de transfert, un récipient à pression, ou un stérilisateur, la température dans ledit récipient étant d'environ 10 °C à environ 40 °C et le récipient comprenant un orifice d'évacuation variable, ledit orifice d'évacuation variable ayant une ouverture d'environ 5 % à environ 95 % de l'ouverture dudit conduit d'évacuation, et ledit niveau de vide initial du récipient étant d'environ 0,01 millibar à environ 15 millibars.

10. Procédé selon la revendication 9, dans lequel ledit récipient (60) est ledit lyophilisateur, dans lequel la température de vaporisation est d'environ 20 °C à environ 150 °C et dans lequel ladite pression partielle de vapeur prédéterminée dudit agent oxydant vaporisé dans ledit récipient est d'environ 7 millibars à environ 50 millibars.

11. Procédé selon la revendication 10, dans lequel ledit agent oxydant est le peroxyde d'hydrogène, l'acide peracétique ou le dioxyde de chlore, ou d'autres biocides comprenant le glutaraldéhyde, l'orthophthaldéhyde ou le formaldéhyde, ou ledit alcool, ou des combinaisons de ces substances, ladite pression de vide initiale étant d'environ 0,01 millibar à environ 0,5 millibar, comprenant un élément chauffant de conduit d'évacuation, l'admission dudit agent oxydant liquide ou de ladite solution oxydante dans ledit vaporiseur (40) se faisant par une pluralité d'impulsions à travers ladite buse d'injection (44), et dans lequel ladite température du récipient est d'environ 15 °C à environ 25 °C.
